(19) Europäisches Patentamt / European Patent Office / Office européen des brevets

(11) **EP 2 570 797 A1**

(12) **EUROPEAN PATENT APPLICATION**
published in accordance with Art. 153(4) EPC

(43) Date of publication:
**20.03.2013 Bulletin 2013/12**

(51) Int Cl.:
***G01N 21/27*** *(2006.01)*

(21) Application number: **11780369.2**

(22) Date of filing: **09.05.2011**

(86) International application number:
**PCT/JP2011/002567**

(87) International publication number:
**WO 2011/142110 (17.11.2011 Gazette 2011/46)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.05.2010 JP 2010109801**

(71) Applicant: **Panasonic Corporation**
**Kadoma-shi**
**Osaka 571-8501 (JP)**

(72) Inventors:
• **TAMURA, Masaya**
**Osaka-shi, Osaka 540-6207 (JP)**

• **KAGATA, Hiroshi**
**Osaka-shi, Osaka 540-6207 (JP)**
• **OKA, Hiroaki**
**Osaka-shi, Osaka 540-6207 (JP)**
• **FUKUSHIMA, Susumu**
**Osaka-shi, Osaka 540-6207 (JP)**

(74) Representative: **Vigand, Philippe et al**
**Novagraaf International SA**
**Chemin de l'Echo 3**
**1213 Onex (CH)**

(54) **PLASMON SENSOR, AND USAGE METHOD AND MANUFACTURING METHOD THEREOF**

(57) A plasmon sensor has a first metal layer and a second metal layer. The first metal layer has a bottom surface and a top surface configured to be supplied with an electromagnetic wave. The second metal layer has a top surface confronting the bottom surface of the first metal layer. Between the first metal layer and the second metal layer, there is provided a hollow region configured to be filled with a specimen containing a medium. Analyte capturing bodies are physically adsorbed at least one of below the first metal layer and above the second metal layer.

FIG. 1

**Description**

TECHNICAL FIELD

The present invention relates to a plasmon sensor using surface plasmon resonance which is usable for sensing of a virus and the like.

BACKGROUND ART

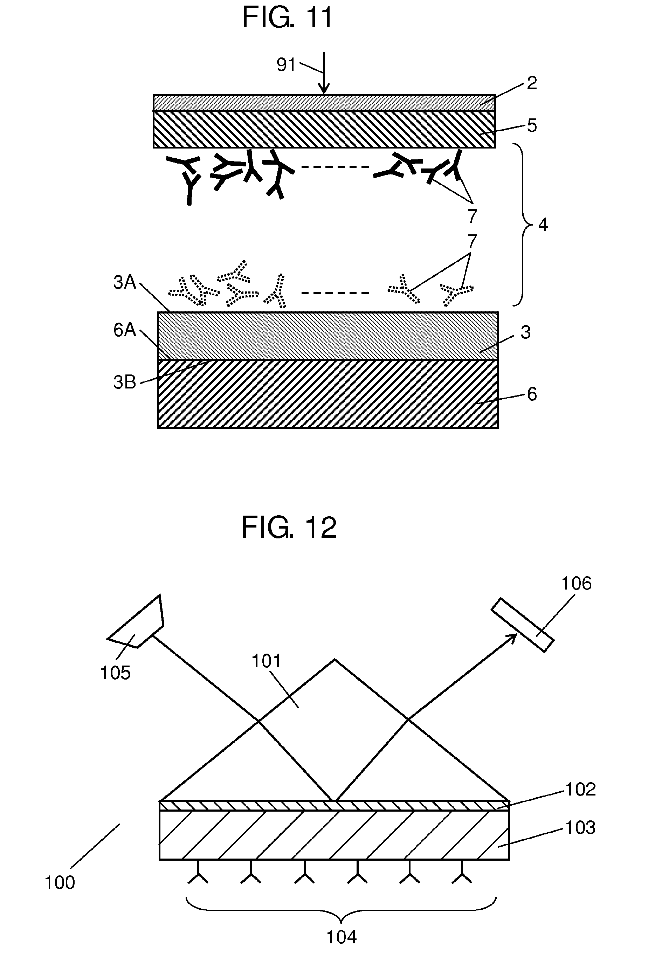

FIG. 12 is a sectional view of plasmon sensor 100 usable for sensing of a virus and the like. Plasmon sensor 100 has prism 101, metal layer 102 with a flat surface, insulating layer 103 with a flat surface and a predetermined dielectric constant, a capturing body 104 as an antibody or the like, light source 105, and wave detecting section 106. Metal layer 102 is disposed on the bottom surface of prism 101, and insulating layer 103 is disposed on the bottom surface of metal layer 102. Capturing body 104 is fixed to the bottom surface of insulating layer 103.

At an interface between metal layer 102 and insulating layer 103, there exists a surface plasmon wave as an electron compressional wave. The surface plasmon wave is an electron wave generated by occurrence of coupling between metal free electrons and light.

Light source 105 is disposed above prism 101. Light source 105 allows p-polarized light to be incident on prism 101 on a total reflection condition. It is to be noted that light oscillating vertically to a plane of incidence is p-polarized light. The light totally reflected on metal layer 102 is received at wave detecting section 106. Wave detecting section 106 detects intensity of the light.

When light source 105 allows light to be incident on prism 101 in such a manner, an evanescent wave is generated at an interface between metal layer 102 and prism 101. The evanescent wave is an electromagnetic wave slightly exuding to the substance side, through which light should not pass, at the time of occurrence of total reflection.

When a wavenumber matching condition in which the wavenumber of the evanescent wave matches up with that of the surface plasmon wave is satisfied here, light energy supplied from light source 105 is used for excitation of the surface plasmon wave, and the intensity of the reflected light decreases. The wavenumber matching condition depends on an incident angle of the light supplied from light source 105. Accordingly, when the incident angle is changed and the intensity of the reflected light is detected at wave detecting section 106, the intensity of the reflected light decreases with a certain incident angle.

An angle at which the intensity of the reflected light is minimal is called a resonance angle. The resonance angle depends on a dielectric constant of insulating layer 103. When an analyte as a substance to be measured in a specimen is specifically coupled with capturing body 104 and the specifically coupled substance is formed on the bottom surface of insulating layer 103, the dielectric constant of insulating layer 103 changes. Accordingly, the resonance angle changes. Therefore, monitoring the change in resonance angle allows sensing of strength and speed of coupling of the specific binding reaction between analyte and capturing body 104, and the like. Such a plasmon sensor is, for example, disclosed in PTL 1.

Plasmon sensor 100 has light source 105 capable of supplying p-polarized light, and prism 101 disposed on the top surface of metal layer 102. For this reason, plasmon sensor 100 has a large, complicated structure.

Citation List

Patent Literature

PTL 1: Unexamined Japanese Patent Publication No. 2005-181296

SUMMARY OF THE INVENTION

The present invention is a small-sized, simply configured plasmon sensor. The plasmon sensor of the present invention has a first metal layer and a second metal layer. The first metal layer has a bottom surface and a top surface configured to be supplied with an electromagnetic wave. The second metal layer has a top surface opposed to the bottom surface of the first metal layer. Between the first metal layer and the second metal layer, there is provided a hollow region configured to be filled with a specimen containing a medium. Analyte capturing bodies physically adsorb to at least one of below of the first metal layer and above of the second metal layer.

In the plasmon sensor of the present invention, a prism is not disposed on the top surface of the first metal layer. Furthermore, even when an electromagnetic wave supplied from an electromagnetic wave source to the first metal layer is not p-polarized, surface plasmon resonance occurs at a first interface between the first metal layer and the hollow region and at a second interface between the second metal layer and the hollow region. It is therefore possible to realize a small-sized, simply configured plasmon sensor. Moreover, the plasmon sensor of the present invention can sense a change in dielectric constant of a substance floating within the hollow region, thus eliminating the need for chemically adsorbing a capturing body such as an antibody to the first metal layer or the second metal layer, for example, via a self-assembled membrane (SAM). It is thus be realizable by a simple process.

Further, for the use of this plasmon sensor, a specimen is inserted into the hollow region with an aid of capillarity, and an electromagnetic wave is supplied to the top surface side of the first metal layer. Then, at least one of a change in amplitude of an electromagnetic wave reflected or radiated from the top surface of the first metal layer and a change in resonance wavelength is sensed.

BRIEF DESCRIPTION OF DRAWINGS

FIG. 1 is a sectional view of a plasmon sensor according to an exemplary embodiment of the present invention.
FIG. 2 is a conceptual view of the plasmon sensor shown in FIG. 1 at the time of inserting a specimen thereinto.
FIG. 3 is a conceptual view of specific binding between a ligand as a capturing body and an analyte.
FIG. 4 is a diagram showing a simulation analysis result of the plasmon sensor according to an exemplary embodiment of the present invention.
FIG. 5A is a conceptual view of a simulation analysis model of the plasmon sensor according to the exemplary embodiment of the present invention.
FIG. 5B is a conceptual view of another simulation analysis model of the plasmon sensor according to the exemplary embodiment of the present invention.
FIG. 6A is a diagram showing a simulation analysis result of the plasmon sensor according to the exemplary embodiment of the present invention.
FIG. 6B is a diagram showing another simulation analysis result of the plasmon sensor according to the exemplary embodiment of the present invention.
FIG. 7 is a sectional view explaining a sensing principle in a plasmon sensor according to the exemplary embodiment of the present invention.
FIG. 8 is a sectional view explaining a sensing principle in a plasmon sensor according to an exemplary embodiment of the present invention.
FIG. 9 is a sectional view explaining a sensing principle in a plasmon sensor according to an exemplary embodiment of the present invention.
FIG. 10 is a sectional view explaining a sensing principle in a plasmon sensor according to an exemplary embodiment of the present invention.
FIG. 11 is a sectional view of another plasmon sensor according to the exemplary embodiment of the present invention.
FIG. 12 is a sectional view of a conventional plasmon sensor.

DESCRIPTION OF EMBODIMENTS

Hereinafter, exemplary embodiments of the present invention will be described with reference to drawings. It is to be noted that in each of the exemplary embodiments, the same constitutional portion as that in a previous exemplary embodiment will be provided with the same reference numeral and its description may be omitted. Further, in the following description, terms indicating directions such as "top surface", "bottom surface", "above", and "below" mean relative directions depending only on a relative positional relation of constitutional components of the plasmon sensor, and do not mean absolute directions, such as a vertical direction.

FIRST EXEMPLARY EMBODIMENT

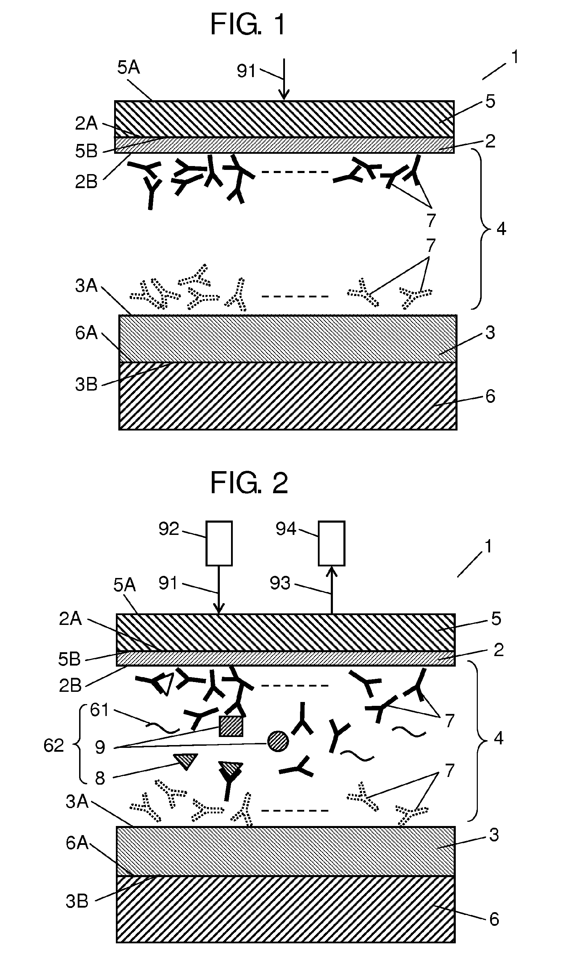

FIG. 1 is a sectional view of plasmon sensor 1 according to a first exemplary embodiment of the present invention. Plasmon sensor 1 has first metal layer (hereinafter, referred to as metal layer) 2, second metal layer (hereinafter, referred to as metal layer) 3, and analyte capturing bodies (hereinafter, referred to as capturing bodies) 7.

Metal layer 2 has bottom surface 2B, and top surface 2A configured to be supplied with an electromagnetic wave. Metal layer 3 has top surface 3A confronting bottom surface 2B of metal layer 2. Hollow region 4 is provided between metal layers 2 and 3. Hollow region 4 is configured to be filled with a specimen containing a medium.

Metal layers 2 and 3 are made of metals such as gold and silver. Since metal layer 2 has a thickness of roughly 100 nm or smaller, it cannot keep its shape alone. Top surface 2A of metal layer 2 is fixed to bottom surface 5B of holding section 5, thereby its shape is maintained. Similarly, metal layer 3 is fixed to top surface 6A of holding section 6, thereby its shape is maintained.

In order to keep a distance between metal layers 2 and 3 constant, plasmon sensor 1 may have a column or a wall which is disposed between metal layers 2 and 3 and holds metal layers 2 and 3. With this structure, plasmon sensor 1 can realize hollow region 4.

Capturing body 7 which is an antibody or the like is physically adsorbed to at least one of bottom surface 2B of metal layer 2 and top surface 3A of metal layer 3. That is, capturing bodies 7 are physically adsorbed to at least one of the below side of metal layer 2 and the above side of metal layer 3. Further, capturing bodies 7 may be disposed, without being oriented, on at least one of the below side of bottom surface 2B of metal layer 2 and the above side of top surface 3A of metal layer 3.

In conventional plasmon sensor 100 shown in FIG. 12, capturing bodies 104 need to be fixed to the bottom surface of insulating layer 103 by chemical adsorption or the like in order to ensure the sensitivity. On the other hand, plasmon sensor 1 has a feature that a resonance wavelength of the surface plasmon changes due to a change in dielectric constant between metal layers 2 and 3. This eliminates the need for fixing capturing body 7 to metal layers 2 and 3 by chemical adsorption or the like. Hence in plasmon sensor 1, it is possible to simplify an arrangement process for capturing bodies 7, such as a SAM membrane formation process, thus manufacturing efficiency is improved. For example, a fluid such as a liquid, a gel, or a gas, containing capturing bodies 7, is injected into hollow region 4 by capillarity, and then dried, thereby to allow arrangement of capturing bodies 7 on at least one of bottom surface 2B of metal layer 2 and top surface 3A of metal layer 3.

FIG. 2 is a conceptual view of plasmon sensor 1 at the time of inserting a specimen. Hollow region 4 can be filled with specimen 62 at the time of using plasmon sensor 1, and hollow region 4 is practically sandwiched between metal layers 2 and 3. Specimen 62 contains analyte 8, antibody 9, and medium 61. Medium 61 is a fluid such as a gas, a liquid, or a gel, and carries analyte 8 and antibody 9.

When hollow region 4 is filled with specimen 62 and analyte 8 in specimen 62 touches capturing body 7, capturing body 7 is specifically coupled with analyte 8. As described above, capturing body 7 is provided below bottom surface 2B of metal layer 2 and/or above top surface 3A of metal layer 3 by physical adsorption. For this reason, capturing body 7 is highly apt to be desorbed as compared with the case of being fixed by chemical adsorption (ion coupling, covalent coupling, or the like). Therefore, when hollow region 4 is filled with specimen 62, a part of physically adsorbed capturing bodies 7 is desorbed and floats in specimen 62. This results in occurrence of specific binding between capturing body 7 and analyte 8 in entire hollow region 4, thereby allowing efficient specific binding between capturing body 7 and analyte 8.

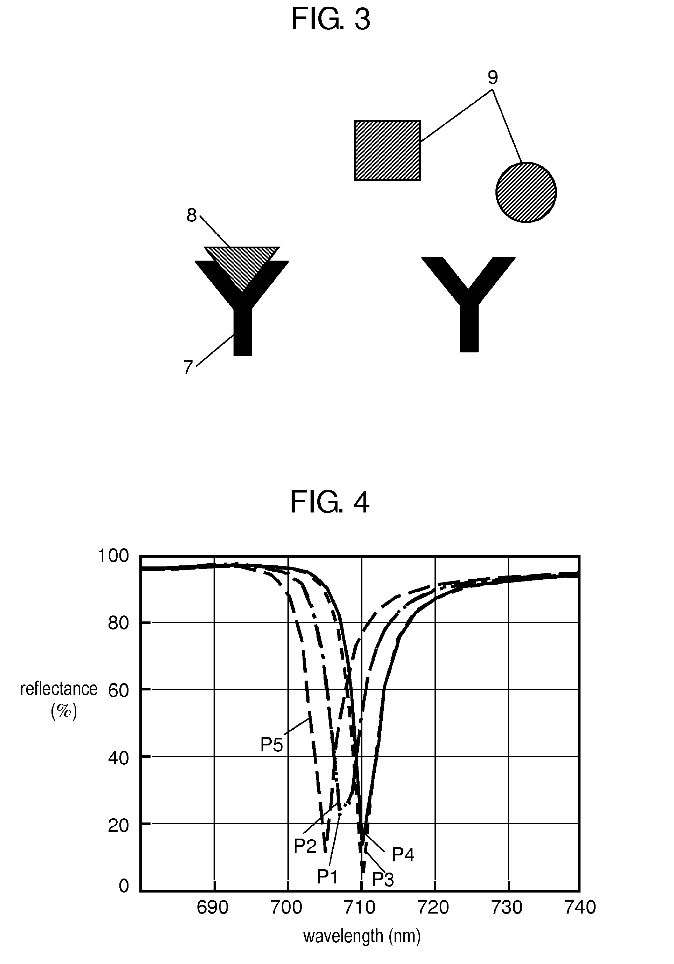

FIG. 3 is a conceptual view showing specific binding between capturing body 7 and analyte 8. Specimen 62 contains antibody 9 as a nonspecific antibody, and analyte 8 as an antibody. Capturing body 7 is not specifically coupled with nonspecific antibody 9, and selectively specifically coupled only with analyte 8.

In FIG. 2, electromagnetic wave source 92 is disposed above top surface 2A of metal layer 2, namely in the direction opposite to metal layer 3 with respect to metal layer 2. Electromagnetic wave source 92 provides electromagnetic wave 91 from the above of top surface 2A of metal layer 2 to metal layer 2.

Hereinafter, an operation of plasmon sensor 1 will be described. In this exemplary embodiment, electromagnetic wave 91 is light and electromagnetic wave source 92 is a light source. Electromagnetic wave source 92 as the light source does not have a device to align polarized waves of light, such as a polarization plate. Unlike plasmon sensor 100 shown in FIG. 12, plasmon sensor 1 can excite surface plasmon resonance not only with p-polarized light, but also with s-polarized light.

Electromagnetic wave 91 provided from the above of metal layer 2 to top surface 2A via holding section 5 is transmitted through metal layer 2, thereafter supplied to hollow region 4, and reaches to top surface 3A of metal layer 3. Due to electromagnetic wave 91, a surface plasmon is generated on bottom surface 2B, as the hollow region 4 side, of metal layer 2, and a surface plasmon is generated on top surface 3A, as the hollow region 4 side, of metal layer 3.

As thus described, even when the electromagnetic wave supplied from electromagnetic wave source 92 to metal layer 2 is not p-polarized, the surface plasmon resonance occurs on the first interface between metal layer 2 and hollow region 4 and the second interface between metal layer 3 and hollow region 4. It is therefore possible to realize plasmon sensor 1 with a small-sized, simple configuration.

When the wavenumber of electromagnetic wave 91 supplied to hollow region 4 matches the wavenumber of the surface plasmon generated on bottom surface 2B of metal layer 2, surface plasmon resonance is excited on bottom surface 2B of metal layer 2. Further, when the wavenumber of electromagnetic wave 91 matches the wavenumber of the surface plasmon generated on top surface 3A of metal layer 3, surface plasmon resonance is excited on top surface 3A of metal layer 3.

In this exemplary embodiment, metal layer 2 has a thickness of roughly 100 nm or smaller. When metal layer 2 has a larger thickness than 100 nm, wavelength components that bring about surface plasmon resonance in the electromagnetic wave (light) are not transmitted through metal layer 2, and hence, surface plasmon resonance is not excited on bottom surface 2B of metal layer 2 and on top surface 3A of metal layer 3. When electromagnetic wave 91 is visible light, the thickness of metal layer 2 made of gold is desirably within a range of 35 nm to 45 nm. With the film thickness being out of this range, surface plasmon resonance is not apt to occur.

The thickness of metal layer 3 made of, for example, gold is desirably not smaller than 100 nm. With a film thickness smaller than 100 nm, incident electromagnetic wave 91 (e.g., visible light) might be transmitted through metal layer 3, and cause deterioration in sensitivity of plasmon sensor 1. That is, when metal layer 3 has a smaller thickness than 100 nm, a part of electromagnetic waves having passed through metal layer 2 and been supplied to hollow region 4 may pass through metal layer 3 and leak to the outside of hollow region 4. As described above, part of energy of the electromagnetic waves to be used for excitation of surface plasmon resonance leaks to the outside of hollow region 4, thereby causing deterioration in sensitivity of plasmon sensor 1. Therefore, making the thickness of metal layer 2 smaller than that of metal layer 3 can enhance the sensitivity of plasmon sensor 1.

A frequency at which surface plasmon resonance occurs is controllable by adjusting at least one of the shapes of metal layers 2 and 3, the distance between metal layers 2 and 3, the dielectric constants of metal layers 2 and 3, a dielectric constant of medium 61 between metal layers 2 and 3, and a distribution of the dielectric constant of medium 61. It is to be noted that in terms of the shapes of metal layers 2 and 3, mainly a change in thickness has a large effect on the frequency at which surface plasmon resonance occurs.

When plasmon sensor 1 receives electromagnetic wave 91 provided from electromagnetic wave source 92, electromagnetic wave 93 is reflected or radiated from plasmon sensor 1. A sensing section 94 for sensing electromagnetic wave 93 is disposed above top surface 2A of metal layer 2, and receives electromagnetic wave 93.

As described above, holding section 5 is fixed to top surface 2A of metal layer 2, and maintains the shape of metal layer 2. Since holding section 5 is required to efficiently supply electromagnetic wave 91 to metal layer 2, it is made of a material not apt to attenuate electromagnetic wave 91. In this exemplary embodiment, with electromagnetic wave 91 being light, holding section 5 is formed of a transparent material, such as glass or transparent plastic, which efficiently transmits light therethrough. The thickness of holding section 5 is preferably as small as possible within a range acceptable in terms of mechanical strength.

With such a structure, it is possible to confine electromagnetic wave 91 as light supplied from electromagnetic wave source 92 in hollow region 4, so as to excite surface plasmon resonance. Further, coupling between the surface plasmon and electromagnetic wave 91 excites the surface plasmon polariton. This excitation leads to absorption of supplied electromagnetic wave 91. The absorbed frequency component is not radiated as electromagnetic wave 93, but another frequency component is radiated as electromagnetic wave 93.

As described above, top surface 6A of holding section 6 is fixed to bottom surface 3B of metal layer 3, and maintains the shape of metal layer 3. In order to enhance the sensitivity of plasmon sensor 1, supplied electromagnetic wave 91 is preferably not transmitted through metal layer 3. Hence, holding section 6 is preferably formed of a material which blocks electromagnetic wave 91. For example, holding section 6 is formed of metal or semiconductor having a thickness of not smaller than 100 nm.

Holding section 6 preferably has a larger thickness than that of holding section 5. This can lead to improvement in mechanical strength of plasmon sensor 1 itself. Consequently, it is possible to prevent deformation and the like of plasmon sensor 1 at the time of the use thereof, and a subsequent change in sensing characteristic thereof.

When the state shown in FIG. 1 is changed to a state where hollow region 4 is filled with specimen 62 as shown in FIG. 2, the dielectric constant between metal layers 2 and 3 (hollow region 4) or a distribution of the dielectric constant between metal layers 2 and 3 changes. This results in a change in resonance wavelength of surface plasmon resonance of plasmon sensor 1. In the following, a comparison will be made between the case of analyte 8 existing in specimen 62 as shown in FIG. 2 and the case of analyte 8 not existing in specimen 62 differently from FIG. 2.

When analyte 8 exists in specimen 62 and specimen 62 is mixed with capturing body 7, specific binding between capturing body 7 and analyte 8 is generated. Molecular structures in the case of separate existence of analyte 8 and capturing body 7 are different from those after specific binding between analyte 8 and capturing body 7. For this reason, after specific binding, the dielectric constant between metal layers 2 and 3 (hollow region 4) changes to a value different from the dielectric constant at the time of separate existence of analyte 8 and capturing body 7. Therefore, the resonance wavelength of plasmon sensor 1 at the time of existence of analyte 8 in the specimen is different from that at the time of non-existence thereof.

FIG. 4 is an analysis result of electromagnetic field simulations, indicating that plasmon sensor 1 has the sensitivity with respect to changes in dielectric constant between metal layers 2 and 3. There will be described the changes in resonance wavelength at the time when the molecular structures after specific binding between capturing body 7 and analyte 8 exists within hollow region 4, with reference to FIG. 4. Specifically, an analysis model for the relation between a position where the molecular structure exists within hollow region 4 after specific binding and the resonance wavelength has the following conditions.

- The molecular structure after specific binding between capturing body 7 and analyte 8 is modeled as a layer of a relative dielectric constant of 1.1 and a thickness of 100 nm.

- Metal layer 2: layer of gold with a thickness of 45 nm
- Metal layer 3: layer of gold with a thickness of 300 nm
- Hollow region 4: a layer of air with a thickness of 1 $\mu$m
- Incident angle of light: vertical direction to top surface 2A of metal layer 2 It is to be noted that CST MW STUDIO is used as an analysis tool in all simulation analyses. Further, a physically adsorbed ligand will not be modeled for the sake of convenience.

Reflectance characteristic curve P5 shown in FIG. 4 indicates a reflectance characteristic in a case where the molecular structure after specific binding between capturing body 7 and analyte 8 does not exist in hollow region 4, and the resonance wavelength is 705.4 nm. Characteristic curve P1 indicates a reflectance characteristic in a case where the molecular structure after specific binding exists on bottom surface 2B of metal layer 2, and the resonance wavelength of plasmon sensor 1 is 707.1 nm. Characteristic curve P2 indicates a reflectance characteristic in a case where the molecular structure after specific binding exists on top surface 3A of metal layer 3, and the resonance wavelength of plasmon sensor 1 is 707.1 nm.

Characteristic curve P3 indicates a reflectance characteristic in a case where the molecular structure after specific binding is disposed on bottom surface 2B of metal layer 2 and top surface 3A of metal layer 3 which border hollow region 4, and the resonance wavelength of plasmon sensor 1 is 710.4 nm. Characteristic curve P4 indicates a reflectance characteristic in a case where the molecular structure after specific binding is disposed in an intermediate position of metal layers 2 and 3, and the resonance wavelength of plasmon sensor 1 is 710.4 nm.

As described above, even when the molecular structure after specific binding between capturing body 7 and analyte 8 exists other than on bottom surface 2B of metal layer 2 and on top surface 3A of metal layer 3, the resonance wavelength of plasmon sensor 1 changes. Taking advantage of this characteristic, it is devised that in plasmon sensor 1, specific binding between capturing body 7 and analyte 8 can be formed not only in regions in the vicinity of metal layers 2 and 3, but in almost entire hollow region 4. For this reason, specific binding can be efficiently formed, resulting in improvement in sensitivity of plasmon sensor 1. Further, in order to allow specific binding to be formed in almost entire hollow region 4, capturing body 7 is disposed within hollow region 4 via physical adsorption by which capturing body 7 is apt to be desorbed. Consequently, the process to provide capturing body 7 within hollow region 4 can be simplified, so as to improve the manufacturing efficiency of plasmon sensor 1.

As described above, plasmon sensor 1 can sense a change in dielectric constant of a substance floating within hollow region 4, thus eliminating the need for chemically adsorbing capturing body 7 to metal layer 2 or metal layer 3, for example, via a self-assembled membrane (SAM). For this reason, it is possible to produce plasmon sensor 1 by a simple process.

Next, a method for using plasmon sensor 1 will be described. First, plasmon sensor 1 will be prepared. Next, specimen 62 is inserted into hollow region 4 with an aid of capillarity as shown in FIG. 2. Electromagnetic wave 91, such as light, will then be incident on (supplied to) the top surface 2A side of metal layer 2 from the top surface 5A side of holding section 5. Then at least one of a change in amplitude of electromagnetic wave 93 reflected or radiated from top surface 2A of metal layer 2 via holding section 5 and a change in resonance wavelength is sensed. Thereby, the existence or non-existence of specific binding within hollow region 4 is checked. For example, sunlight or fluorescent light is incident from the top surface 5A side of holding section 5, and a change in color of reflected light thereto is sensed with human eyes, thus it can be checked whether or not specific binding exists within hollow region 4.

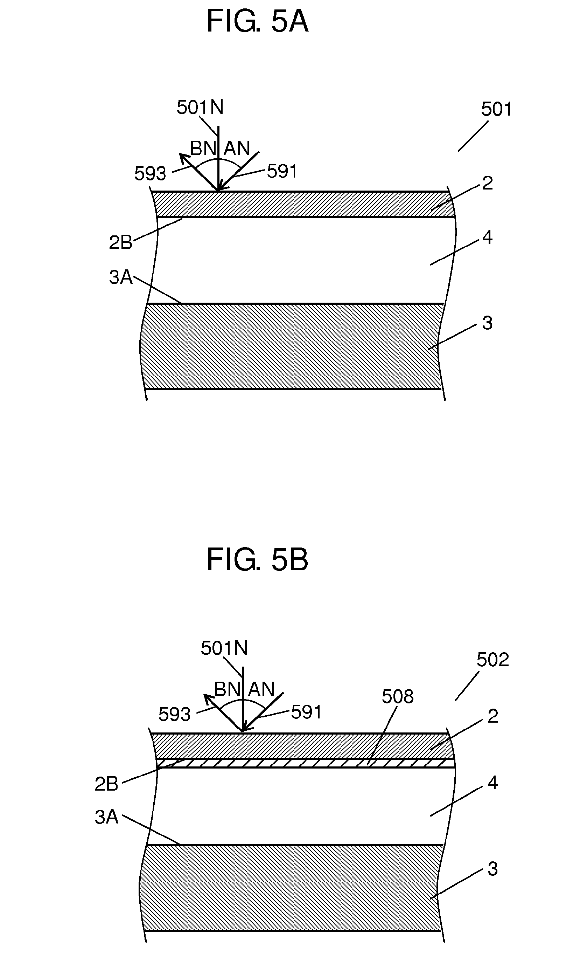

Hereinafter, a specific principle thereof will be described. In plasmon sensor 1, at a resonance frequency of the surface plasmon, the electromagnetic field intensity between metal layers 2 and 3 may be distributed on a higher order mode. That is, the intensity of the electromagnetic field generated between metal layers 2 and 3 may be locally larger in a plurality of places. The state thereof will be described using analysis models 501 and 502 of an electromagnetic field simulation shown in FIGS. 5A and 5B.

In analysis model 501, metal layer 2 is made of silver, and has a thickness of 30 nm. Metal layer 3 is made of silver, and has a thickness of 130 nm. The distance between metal layers 2 and 3 is 10 $\mu$m, and hollow region 4 is filled with air having a relative dielectric constant of 1. The above of top surface 2A of metal layer 2 and the below of bottom surface 3B of metal layer 3 are filled with air. In analysis model 501, metal layers 2, 3 and hollow region 4 unlimitedly continue in a transverse direction.

In analysis model 502, a resultant substance 508 obtained from specific binding between capturing body 7 and analyte 8 is disposed on bottom surface 2B of metal layer 2 in analysis model 501 shown in FIG. 5A. Resultant substance 508 has a thickness of 10 nm, and a relative dielectric constant of 3.0.

A dielectric function of silver constituting metal layers 2 and 3 can be produced by converting experimental data on refractive index, described in "Handbook of Optical Constants of Solids"(Palik, Edward D. in 1998). In analysis models 501 and 502, capturing body 7 is not modeled in order that a simple simulation analysis can be performed.

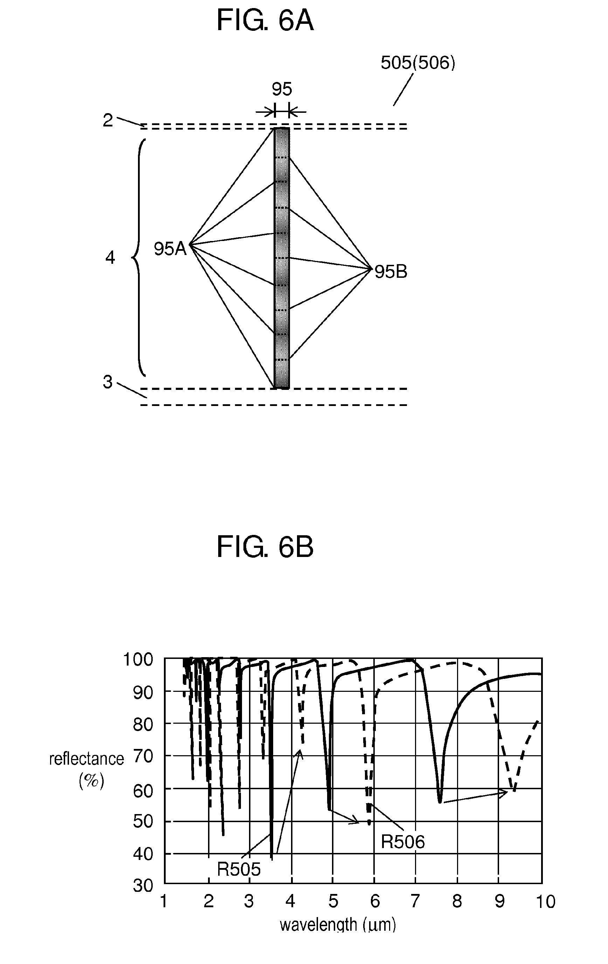

Electromagnetic wave 591 is provided from elevation angle AN of 45 degrees with respect to normal line direction 501N of top surface 2A of metal layer 2, and electromagnetic wave 593 radiated from top surface 2A of metal layer 2 at elevation angle BN of -45 degrees is sensed. FIGS. 6A and 6B show a result of the electromagnetic field simulation analysis performed based on the above conditions.

FIG. 6A shows an electromagnetic field simulation result of analysis model 501. The resonance wavelength of this model is 2883 nm, and FIG. 6A represents a distribution of the electric field intensity of hollow region 4 by shading. It is to be noted that FIG. 6A does not show an electric field distribution in every region of hollow region 4, but only represents an electric field distribution in certain region 95, for the sake of description.

The electric field intensity that exists between metal layers 2 and 3 periodically repeats a local change in a position from metal layer 2 toward metal layer 3, and the electric field intensity is smaller in regions in the vicinity of metal layers 2 and 3. In FIG. 6A, the electric field intensity is locally larger in a plurality of, namely five, regions 95A between metal layers 2 and 3, and is locally smaller in regions 95B therebetween. In region 95A, the electromagnetic field intensity is distributed on a higher order mode than a fundamental mode.

By distribution of the electromagnetic field intensity between metal layers 2 and 3 on a higher order mode, a space between metal layers 2 and 3 can be expanded, so as to facilitate insertion of specimen 62 containing analyte 8 into hollow region 4.

In analysis model 505 shown in FIG. 6A, the relative electric constant of hollow region 4 is 1. FIG. 6B shows reflectance characteristics R505 and R506 as a result of electromagnetic field simulations of analysis model 505 and analysis model 506 having a relative dielectric constant of 1.2 in the hollow region of analysis model 505.

In FIG. 6B, a horizontal axis indicates a wavelength of electromagnetic wave 591, and a vertical axis indicates a reflectance as a ratio of electric power of electromagnetic wave 593 with respect to electric power of electromagnetic wave 591. Reflectance characteristics R505 and R506 show that surface plasmon resonance occurs at a large number of resonance wavelengths in analysis models 505 and 506. Further, those show that the resonance wavelength changes by changing the state of medium in hollow region 4, namely a relative dielectric constant. As described above, in the case of making hollow region 4 thick, the electromagnetic field intensity is distributed on a higher order mode between metal layers 2 and 3, and surface plasmon resonance occurs at a higher order frequency.

In plasmon sensor 1, a change over time in state of medium 61 in hollow region 4 can be sensed using surface plasmon resonance that occurs at a higher order mode frequency. This enables expansion of the space between metal layers 2 and 3, so as to facilitate insertion of specimen 62 containing analyte 8 into hollow region 4.

Next, there will be described a method for deriving an order of a higher order mode in plasmon sensor 1. When the electromagnetic field intensity between metal layers 2 and 3 is distributed on an "m"-order mode before specimen 62 having refractive index "n" and not containing analyte 8 is disposed in hollow region 4, equation 1 holds, using integer "a" of 1 or larger.

$$(1/2) \times \lambda \times \mathrm{m} = (1/2) \times (\lambda/\mathrm{n}) \times (\mathrm{m} + \mathrm{a}) \quad \text{(Equation 1)}$$

In equation 1, "λ" is a wavelength of electromagnetic wave 91 in hollow region 4, which is supplied from the above of top surface 2A of metal layer 2, before medium 61 is disposed in hollow region 4.

The left-hand side of equation 1 shows the distance between metal layers 2 and 3 before medium 61 is disposed in hollow region 4. That is, since the electromagnetic field intensity is distributed on an "m"-order mode between metal layers 2 and 3 before medium 61 is disposed in hollow region 4, the distance between metal layers 2 and 3 is represented by the left-hand side of equation 1.

The right-hand side of equation 1 shows the distance between metal layer 2 and metal layer 3 after medium 61 is disposed in hollow region 4. That is, when medium 61 having refractive index n is disposed in hollow region 4, wavelength "λ" of electromagnetic wave 91 in hollow region 4 is reduced into 1/n. Hence, a large number of nodes and antinodes in the electromagnetic field intensity are generated between metal layers 2 and 3 as compared with those before medium 61 is disposed. When the electromagnetic field intensity is distributed on an (m + a) order mode at this time, the distance between metal layers 2 and 3 is represented by the right-hand side of equation 1. The left-hand side and the right-hand side of equation 1 both represent the distance between metal layers 2 and 3, and are thus equal. Integer "a" represents a difference in order of the mode in the distribution of the electromagnetic field intensity which changes in accordance with the existence or non-existence of medium 61 (specimen 62 not containing analyte 8) between metal layers 2 and 3.

From equation 1, order "m" of the higher order mode, refractive index "n" and integer "a" satisfy equation 2.

$$m = a/(n-1) \quad (\text{Equation } 2)$$

The change in resonance wavelength of plasmon sensor 1 can be sensed with user's eyes. That is, the change in resonance wavelength can be sensed from a color of reflected light from plasmon sensor 1. In order to determine whether or not specimen 62 contains analyte 8, the following conditions need to be met. That is, when only medium 61 as specimen 62 not containing analyte 8 is disposed in hollow region 4, the color of reflected light from plasmon sensor 1 does not change. Only when specimen 62 containing analyte 8 is disposed in hollow region 4, the color of reflected light changes. For this reason, it is necessary to prevent a change in color of reflected light from plasmon sensor 1 in accordance with whether or not specimen 62 not containing analyte 8, namely medium 61, is disposed in hollow region 4.

For example, order "m" is obtained as follows when specimen 62 not containing analyte 8, namely medium 61, is water. Refractive index "n" of water is 1.3334. When integer "a" is set to 1, m = 2.9994 ≅ 3 from equation 2.

A visible light band is a wavelength band of light visible with human eyes, and in a range of wavelengths from 380 nm to 750 nm, inclusive. Herein, for example, plasmon sensor 1 is designed so as to make surface plasmon resonance occur at frequency "fb" within a blue wavelength band from 450 nm to 495 nm as the visible light band.

In a state where water is not disposed in hollow region 4, namely a state where air is disposed therein, the distance between metal layers 2 and 3 is decided such that an electromagnetic field distribution on a third-order mode occurs at frequency "fb" in hollow region 4. The third-order mode is selected because the above calculation result is m ≅ 3.

In plasmon sensor 1, surface plasmon resonance occurs roughly at frequency "fb". When white light containing frequency compounds throughout the visible light band is incident on top surface 2A of metal layer 2, it is reflected on top surface 2A, and the reflected light is emitted upward. In this reflected light, blue light is particularly attenuated within the incident white light.

Next, when specimen 62 which does not contain analyte 8 but is only water as medium 61 is disposed in hollow region 4, the electromagnetic field is distributed roughly on a fourth-order mode (m + a = 2.9994 + 1 ≅ 4) at frequency fb between metal layers 2 and 3. That is, even when medium 61 is disposed in hollow region 4, surface plasmon resonance occurs at frequency "fb" in plasmon sensor 1, and hence the color of light reflected toward the above of metal layer 2 roughly does not change. It is thereby possible to prevent large shift of the resonance wavelength of plasmon sensor 1 in accordance with whether or not only medium 61 is disposed in hollow region 4.

It should be noted that, although "m" is approximate to 3 on the above condition, derived "m" is rarely an integer, and hence an integer value obtained by rounding off the value of derived "m" is set as integer "m".

Further, the distance between metal layers 2 and 3 may be designed such that, when the state is changed from one where medium 61 is not disposed in hollow region 4 to one where only medium 61 is disposed in hollow region 4, the wavelength at which surface plasmon resonance occurs changes only within a specific wavelength band. Specifically, the order of the mode in distribution of the electromagnetic field generated between metal layers 2 and 3 is set in a similar manner to the above.

Examples of these specific wavelength bands include wavelength band A, wavelength band B, wavelength band C, wavelength band D, wavelength band E, and wavelength band F. Wavelength band A is not smaller than 380 nm and smaller than 450 nm, wavelength band B is not smaller than 450 nm and smaller than 495 nm, wavelength band C is not smaller than 495 nm and smaller than 570 nm, wavelength band D is not smaller than 570 nm and smaller than 590 nm, wavelength band E is not smaller than 590 nm and smaller than 620 nm, and wavelength band F is not smaller than 620 nm and smaller than 750 nm.

Wavelength band A is a wavelength band corresponding to purple in the visible light band, wavelength band B is a wavelength band corresponding to blue in the visible light band, and wavelength band C is a wavelength band corresponding to green in the visible light band. Wavelength band D is a wavelength band corresponding to yellow in the visible light band, wavelength band E is a wavelength band corresponding to orange in the visible light band, and wavelength band F is a wavelength band corresponding to red in the visible light band. By the change in wavelength of reflected light within one wavelength band among these wavelength bands, it is possible to prevent a large change in color of reflected light from plasmon sensor 1 in accordance with the existence or non-existence of specimen 62 without analyte 8. That is, it is possible by human vision to easily sense only the existence or non-existence of analyte 8, so as to sense an antigen-antibody reaction.

It is to be noted that hollow region 4 may be provided in roughly entire region between metal layers 2 and 3 (including a region not provided with capturing body 7). Further, hollow region 4 may be provided in a region (including the region not provided with capturing body 7) other than a column or a wall which supports metal layers 2 and 3 between metal layers 2 and 3. Moreover, a corrosion-prevention coating layer may be applied to bottom surface 2B of metal layer 2 and top surface 3A of metal layer 3. In that case, hollow region 4 may be provided in a region other than the corrosion-prevention coating layer between metal layers 2 and 3. However, the region of capturing body 7 disposed on the surface of the corrosion-prevention coating agent, which is not in contact with metal layer 2 or metal layer 3, is not included. A region that can be inserted with specimen 62 is hollow region 4, and hollow region 4 may be ensured in a part of the region between metal layers 2 and 3.

Space L between metal layers 2 and 3 is represented in equation 3 below by frequency F at which surface plasmon resonance occurs.

$$L = N \times C/(2 \times F) \times \cos\theta \quad (\text{Equation } 3)$$

In equation 3, N is a counting number, C is an effective light speed between metal layers 2 and 3, and θ is an incident angle of an electromagnetic wave with respect to a normal line vertical to bottom surface 2B of metal layer 2 and top surface 3A of metal layer 3 in hollow region 4. It is to be noted that equation 3 includes an error since complex refraction indexes of metal layers 2 and 3 are not considered therein. When a medium other than hollow region 4 (such as the foregoing column or wall) exists between metal layers 2 and 3, the value of C in equation 3 is a value obtained in consideration of such a medium.

An electromagnetic wave transmitted through metal layer 2 and entered into hollow region 4 is reflected on top surface 3A of metal layer 3. This results in occurrence of standing distribution of the electromagnetic field intensity in hollow region 4, as shown in FIG. 6A. Surface plasmon resonance occurs using a part of the electromagnetic field subjected to standing distribution occurred in hollow region 4, as an energy source.

Plasmon sensor 1 may be designed such that the state of medium 61 in hollow region 4 is temporally changed, which leads to a change in resonance wavelength from an invisible light band as a wavelength band other than the visible light band to a visible light band, or a change from the visible light band to the invisible light band.

For example, when the state of medium 61 in hollow region 4 changes due to specific binding between capturing body 7 and analyte 8, the resonance wavelength may change from the invisible light band to the visible light band. In this case, part of the color of light in the visible light band which can be sensed with human eyes is not apt to be reflected or radiated from plasmon sensor 1 due to surface plasmon resonance. Consequently, it becomes possible to sense specific binding between capturing body 7 and analyte 8 with human eyes, so that simple plasmon sensor 1 not including a complicated large-scaled device can be realized.

In the foregoing description, electromagnetic wave 91 supplied to plasmon sensor 1 contains at least a wavelength of part of the visible light band. Specifically, sunlight or illuminated light as white light is applied to plasmon sensor 1, and its reflected light or radiated light can be sensed with human eyes. This can facilitate sensing of specific binding and the like between capturing body 7 and analyte 8 with human eyes.

When an angle at which electromagnetic wave 91 is supplied to plasmon sensor 1 (e.g., incident angle of electromagnetic wave 91 on metal layer 2) changes, a resonance wavelength also changes. For this reason, particular attention needs to be paid to design of plasmon sensor 1 when the incident angle of electromagnetic wave 91 that is incident on plasmon sensor 1 changes. That is, plasmon sensor 1 needs to designed such that the resonance wavelength falls within the region of the invisible light band even when an angle at which electromagnetic wave 91 is supplied to plasmon sensor 1 is changed in a possible range in a state before occurrence of specific binding. Alternatively, it needs to be designed such that the resonance wavelength falls within a wavelength band region of the same color in the visible light band. By designing the plasmon sensor 1 in such manners, the color of reflected light remains unchanged even when a supply angle of the electromagnetic wave to plasmon sensor 1 is changed in a possible range in the case of holding plasmon sensor 1 with hands and applying sunlight to the metal layer 2 side to sense specific binding between capturing body 7 and analyte 8. Materials for holding sections 5 and 6, thicknesses of and materials for metal layers 2 and 3, the distance between metal layers 2 and 3 and the like are adjusted so that plasmon sensor 1 is designed as described above.

In the foregoing description, the resonance wavelength of plasmon sensor 1 is changed from the invisible light band to the visible light band, or changed from the visible light band to the invisible light band. Plasmon sensor 100 shown in FIG. 12 may be designed such that the above change occurs in plasmon sensor 100. Specifically, it is configured such that the resonance wavelength of plasmon sensor 100 having prism 101 shown in FIG. 12 is changed from the invisible light band to the visible light band, or changed from the visible light band to the invisible light band, before and after specific binding between capturing body 104 and the analyte. Further, a similar design concept may be applied to a sensor using the localized plasmon. This can facilitate sensing of specific binding between the capturing body and the analyte, and the like, with human eyes.

Further, plasmon sensor 1 may be designed such that, by temporally changing the state of medium 61 in hollow region 4, the wavelength at which surface plasmon resonance occurs changes from the invisible light band to the wavelength band of blue to green light or the wavelength band of red light. The wavelength band of blue light is not smaller than 450 nm and smaller than 495 nm, and the wavelength band of green light is from 495 nm to 570 nm, inclusive. Therefore, the former wavelength band is from 450 nm to 570 nm, inclusive. The wavelength band of red light is from 620 nm to 750 nm, inclusive. Alternatively, plasmon sensor 1 may be designed such that the wavelength at which surface plasmon resonance occurs changes from either of these two wavelength bands to the invisible light band.

A cone cell densely distributed to the midsection of a retina of a human is formed of three kinds of cones, which are a cone to absorb red light, another cone to absorb green light, and further another cone to absorb blue light. Thus, light which can be sensed by the human are light of only three colors, which are red, blue and green. As described above, making use of blue, green and red light, to which human eyes have extremely high sensitivities, can facilitate sensing of a change in electromagnetic wave (light) from plasmon sensor 1 by human vision.

For example, the state of the medium in hollow region 4 changes due to specific binding between capturing body 7 and analyte 8, and the resonance wavelength changes from the invisible light band to the region from 450 nm to 570 nm, inclusive, or the region from 620 nm to 750 nm, inclusive. The color of one light among blue, green and red, to which the human vision has the highest sensitivity, is then not apt to be reflected or radiated from plasmon sensor 1 due to surface plasmon resonance. This results in highly sensitive sensing of specific binding and the like between capturing body 7 and analyte 8, with human eyes.

Also in this case, when the supply angle of electromagnetic wave 91 to plasmon sensor 1 (e.g., incident angle of the electromagnetic wave on metal layer 2) changes, the resonance wavelength also changes. For this reason, particular attention needs to be paid to design of plasmon sensor 1 when the incident angle of electromagnetic wave 91 that is incident on plasmon sensor 1 changes. That is, it needs to be designed such that the resonance wavelength falls within the region of the invisible light band even when an angle at which electromagnetic wave 91 is supplied to plasmon sensor 1 is changed in a possible range in a state before occurrence of specific binding. Alternatively, it needs to be designed such that the resonance wavelength falls within a wavelength band region of the same color in the visible light band. By designing the plasmon sensor 1 in such manners, the color of reflected light remains unchanged even when a supply angle of the electromagnetic wave to plasmon sensor 1 is changed in a possible range in the case of holding plasmon sensor 1 with hands and applying sunlight to the metal layer 2 side to sense specific binding between capturing body 7 and analyte 8.

It is preferable that the electromagnetic wave to be supplied to plasmon sensor 1 contains at least wavelengths of blue, green and red light. This allows sensing of specific binding and the like between capturing body 7 and analyte 8 with human eyes as described above.

Further, in the foregoing description, the resonance wavelength of plasmon sensor 1 is changed from the invisible light band to the region from 450 nm to 570 nm, inclusive, or the region from 620 nm to 750 nm, inclusive, or is changed from the region from 450 nm to 570 nm, inclusive, or the region from 620 nm to 750 nm, inclusive, to the invisible light band. This change may be applied to conventional plasmon sensor 100. Specifically, plasmon sensor 100 can be configured such that the resonance wavelength of plasmon sensor 100 having prism 101 shown in FIG. 12 is changed from the invisible light band to the region from 450 nm to 570 nm, inclusive, or the region from 620 nm to 750 nm, inclusive, or is changed from the region from 450 nm to 570 nm, inclusive, or the region from 620 nm to 750 nm, inclusive, to the invisible light band, before and after specific binding between capturing body 104 and the analyte. Further, this change may be applied to a sensor using the localized plasmon. This can facilitate sensing of specific binding and the like between the capturing body and the analyte with human eyes easily.

Further, plasmon sensor 1 may be designed such that, by temporally changing the state of medium 61 in hollow region 4, the wavelength at which surface plasmon resonance occurs changes from the region of not smaller than 450 nm and smaller than 495 nm to the region from 495 nm to 580 nm, inclusive.

As a specific example, plasmon sensor 1 is supposed, from which reflected light or radiated light is sensed with human eyes when sunlight or illuminated light containing a large number of visible light rays enters from the above of top surface 2A of metal layer 2 of plasmon sensor 1. Surface plasmon resonance occurs at the wavelength of not smaller than 450 nm and smaller than 495 nm, which corresponds to blue light, before the change in medium 61 in hollow region 4 of plasmon sensor 1. Hence an electromagnetic wave (light), obtained by weakening only blue light corresponding to the resonance wavelength from sunlight or illuminated light containing a large number of visible light rays, is reflected or radiated from plasmon sensor 1. The human recognizes such an electromagnetic wave (light).

Next, surface plasmon resonance occurs at the wavelength from 495 nm to 580 nm, inclusive, which corresponds to green light, after the change in medium 61 in hollow region 4 of plasmon sensor 1. Hence an electromagnetic wave (light), obtained by weakening only green light corresponding to the resonance wavelength from sunlight or illuminated light containing a large number of visible light rays, is reflected or radiated from plasmon sensor 1. The human recognizes such an electromagnetic wave (light). Since the human eyes have high sensitivities to green and blue light, it is possible to easily recognize with the eyes that the resonance wavelength has changed from the blue light region to the green light region due to the change in medium 61 in hollow region 4. Hence it is possible to sense a change in plasmon sensor 1 only by human vision, even without use of specific equipment as electromagnetic wave source 92 or sensing section 94.

Further, as the blue and green light wavelengths are adjacent to each other, it is possible to make small an amount of change in resonance wavelength due to the change in medium 61 in hollow region 4. Hence plasmon sensor 1 with this configuration is usable even in the case of analyte 8 or the like having a low relative dielectric constant.

In addition, although the example using sunlight or illuminated light is shown as the electromagnetic wave in the foregoing description, this is not restrictive, and the electromagnetic wave may contain at least blue and green lights.

Although the case has been shown where the resonance wavelength of plasmon sensor 1 is changed from the region of not smaller than 450 nm and smaller than 495 nm to the region from 495 nm to 580 nm, inclusive, in the foregoing description, this design concept may be applied to conventional plasmon sensor 100, and the like. Specifically, the resonance wavelength of plasmon sensor 100 having prism 101 shown in FIG. 12 may be changed from the region of not smaller than 450 nm and smaller than 495 nm to the region from 495 nm to 580 nm, inclusive, before and after specific binding between capturing body 104 and the analyte. Further, a similar concept may be applied to a sensor using the localized plasmon. This can facilitate sensing of specific binding and the like between the capturing body and the analyte with human eyes.

Further, plasmon sensor 1 may be designed such that, by temporally changing the state of medium 61 in hollow region 4, the wavelength at which surface plasmon resonance occurs changes from any of forgoing wavelength band A, wavelength band B, wavelength band C, wavelength band D, wavelength band E, and wavelength band F, to another wavelength band. Wavelength band A is not smaller than 380 nm and smaller than 450 nm, wavelength band B is not smaller than 450 nm and smaller than 495 nm, and wavelength band C is not smaller than 495 nm and smaller than 570 nm. Wavelength band D is not smaller than 570 nm and smaller than 590 nm, wavelength band E is not smaller than 590 nm and smaller than 620 nm, and wavelength band F is not smaller than 620 nm and smaller than 750 nm. Specifically, such a design can be realized by means of the space between metal layer 2 and metal layer 3, the thickness of metal layer 2, and the like, in plasmon sensor 1.

When the state of the medium in hollow region 4 temporally changes, although the resonance wavelength has been within one of wavelength bands A to F before specific binding, it moves into another band after specific binding. Specifically, the wavelength band of the resonance wavelength moves when capturing body 7 is specifically coupled to analyte 8 in hollow region 4. This can facilitate sensing of specific binding and the like between capturing body 7 and analyte 8 with human eyes.

In the foregoing description, the resonance wavelength of plasmon sensor 1 is changed from one wavelength among wavelength bands A to F band to another wavelength band. This change may be applied to conventional plasmon sensor 100. Specifically, the resonance wavelength of plasmon sensor 100 having prism 101 shown in FIG. 12 may be changed from any one wavelength band of wavelength bands A to F to another wavelength band before and after specific binding between capturing body 104 and the analyte. Further, a similar change may be applied to a sensor using the localized plasmon. This can facilitate sensing of specific binding and the like between the capturing body and the analyte with human eyes.

Further, plasmon sensor 1 may be configured such that the wavelength at which surface plasmon resonance occurs changes from the invisible light band to any wavelength band of wavelength bands A to F by temporally changing the state of medium 61 in hollow region 4. Alternatively, it may be configured such that the wavelength band changes from any wavelength band of wavelength bands A to F to the invisible light band.

When the state of medium 61 in hollow region 4 temporally changes, in at least one of states before and after the change, reflected light in any one of wavelength bands A to F (reflected light from plasmon sensor 1) attenuates due to surface plasmon resonance. This can facilitate sensing of specific binding and the like between capturing body 7 and analyte 8 with human eyes.

In the foregoing description, the resonance wavelength of plasmon sensor 1 is changed from the invisible light band to any one of wavelength bands A to F, or changed from any one of wavelength bands A to F to the invisible light band. This design concept may be applied to conventional plasmon sensor 100. Specifically, the resonance wavelength of plasmon sensor 100 having prism 101 shown in FIG. 12 may be changed from the invisible light band to any one of wavelength bands A to F before and after specific binding between capturing body 104 and the analyte. Alternatively, the wavelength band may be changed from any one of wavelength bands A to F to the invisible light band. Moreover, a plasmon sensor using the localized plasmon may be designed so as to bring about a change in wavelength of the reflected light. This can facilitate sensing of specific binding between the capturing body and the analyte with human eyes.

When conventional plasmon sensor shown in FIG. 12 is held by the human in the hand, a portion where surface plasmon resonance occurs, namely a portion disposed with capturing body 104, is undesirably touched by the human in the hand, thereby the resonance frequency changes. On the other hand, in plasmon sensor 1, a portion where surface plasmon resonance occurs is at least one of bottom surface 2B of metal layer 2 which borders hollow region 4 and top surface 3A of metal layer 3 which borders hollow region 4. This portion is difficult to directly touch with the hand. For this reason, the resonance frequency is not apt to change even the sensor is used by the human as being held in the hand.

Next, an example of a method for manufacturing plasmon sensor 1 will be described. First, metal layer 2 is formed on bottom surface 5B of holding section 5 formed of a transparent resin, glass or the like. Meanwhile, metal layer 3 is formed on top surface 6A of holding section 6 formed of metal, semiconductor, or the like. Metal layers 2 and 3 can be formed by sputtering, for example; however, a formation method thereof is not particularly restricted. Next, holding sections 5 and 6 are disposed such that hollow region 4 can be provided between metal layer 2 and metal layer 3. In such a manner, a plasmon sensor structure is prepared where metal layer 2 having bottom surface 2B and top surface 2A configured to be supplied with an electromagnetic wave, and metal layer 3 having top surface 3A confronting bottom surface 2B of metal layer 2, are provided and hollow region 4 is provided between metal layer 2 and metal layer 3.

Subsequently, a medium containing capturing body 7 is inserted into hollow region 4 with an aid of capillarity. That is, a solution, slurry, an emulsion or the like, containing capturing body 7, is inserted into hollow region 4. Thereafter, the inserted medium is dried so as to dispose capturing body 7 in at least one of below metal layer 2 and above metal layer 3.

In plasmon sensor 1, capturing body 7 does not need to be fixed within hollow region 4 by chemical adsorption or the like. For this reason, after combination of metal layer 2 with metal layer 3 via a column or the like for ensuring and keeping hollow region 4, capturing body 7 can be disposed within hollow region 4 by a simple method as above. This can improve efficiency in manufacturing plasmon sensor 1.

SECOND EXEMPLARY EMBODIMENT

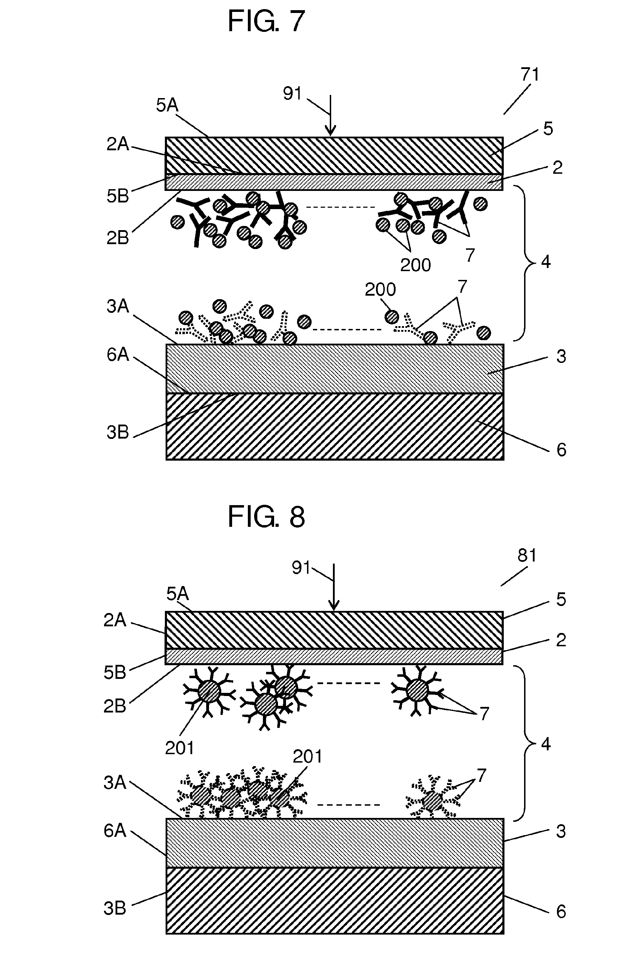

FIG. 7 is a sectional view of plasmon sensor 71 according to a second exemplary embodiment of the present invention. Plasmon sensor 71 differs from plasmon sensor 1 in the first exemplary embodiment in that capturing bodies 7 are physically adsorbed to at least one of bottom surface 2B of metal layer 2 and top surface 3A of metal layer 3, together with additive 200. Disposing additive 200 around capturing body 7 can prevent denaturalization of capturing body 7 due to the effect of drying and the like. Furthermore, when specimen 62 shown in FIG. 2 is inserted into hollow region 4, additive 200 acts on capturing body 7. For this reason, desorption of capturing body 7 is promoted, thus analyte 8 and capturing body 7 make the specific binding therebetween efficiently within hollow region 4.

Furthermore, adopting appropriate additive 200 such as polyethylene glycol and phosphorylcholine can also improve an infusion rate of specimen 62 into hollow region 4 by capillarity. This can result in improvement in detection efficiency. Further, it is also possible to prevent bubbles from remaining between capturing body 7 and capturing body 7 adjacent thereto after infusion of specimen 62.

Plasmon sensor 71 can be manufactured, and can also be used, in similar manners to plasmon sensor 1 in the first exemplary embodiment. Plasmon sensor 71 also has similar advantageous effects to plasmon sensor 1 in the first exemplary embodiment.

THIRD EXEMPLARY EMBODIMENT

FIG. 8 is a sectional view of plasmon sensor 81 according to a third exemplary embodiment of the present invention. Plasmon sensor 81 differs from plasmon sensor 1 in the first exemplary embodiment in that capturing bodies 7 are chemically adsorbed to the surface of particle 201. Particle 201 may be made of metal material, magnetic material, dielectric material, rubber, or the like as an inorganic material, or may be made of a dendrimer as an organic material. As a method for chemical adsorption, a method of fixing capturing bodies 7 to particle 201 via a self-assembled monolayer can be considered, for example.

Fixing capturing bodies 7 to particle 201 and holding it allows each capturing body 7 to easily come into contact with analyte 8. Therefore, capturing body 7 and analyte 8 can efficiently make the specific binding therebetween.

In the case of using metal material (e.g., gold colloid) as particle 201, adjusting a size of particle 201 can lead to occurrence of localized plasmon resonance on the surface of particle 201. Therewith, an electromagnetic wave component of the resonance wavelength of the localized plasmon generated on the surface of particle 201 is not apt to be emitted from plasmon sensor 81 to the outside. When capturing body 7 fixed to the surface of particle 201 is specifically coupled with analyte 8, the dielectric constant of the surface of particle 201 changes. For this reason, the resonance wavelength of the localized plasmon changes. As this phenomenon also can be used to check the existence or non-existence of an antigen-antibody reaction, the sensitivity of plasmon sensor 81 is improved.

When magnetic material having a property of being attracted to a magnet is used as particle 201 and a magnetic field is applied from the outside of plasmon sensor 81 after infusion of specimen 62 shown in FIG. 2 into hollow region 4, particle 201 fixed with capturing body 7 can be stirred. This enables efficient specific binding between capturing body 7 and analyte 8.

On the other hand, since the shape of the dendrimer can be made uniform, variations in shape of particle 201 can be reduced in the case of using the dendrimer as particle 201. This can lead to realization of uniform plasmon resonance in plasmon sensor 81.

Similarly to plasmon sensor 71 of the second exemplary embodiment, plasmon sensor 81 may be configured such that additive 200 is disposed around particle 201. This allows plasmon sensor 81 to also exert similar advantageous effects to plasmon sensor 71 in the second exemplary embodiment.

Further, plasmon sensor 81 can be manufactured, and can also be used, in similar manners to plasmon sensor 1 in the first exemplary embodiment. Further, plasmon sensor 81 also has similar advantageous effects to plasmon sensor 1 in the first exemplary embodiment.

It is to be noted that, although particle 201 is shown in spherical form in FIG. 8 for the sake of convenience, even when one having a tridimensional shape other than this is used, a similar effect to the above can be obtained.

FOURTH EXEMPLARY EMBODIMENT

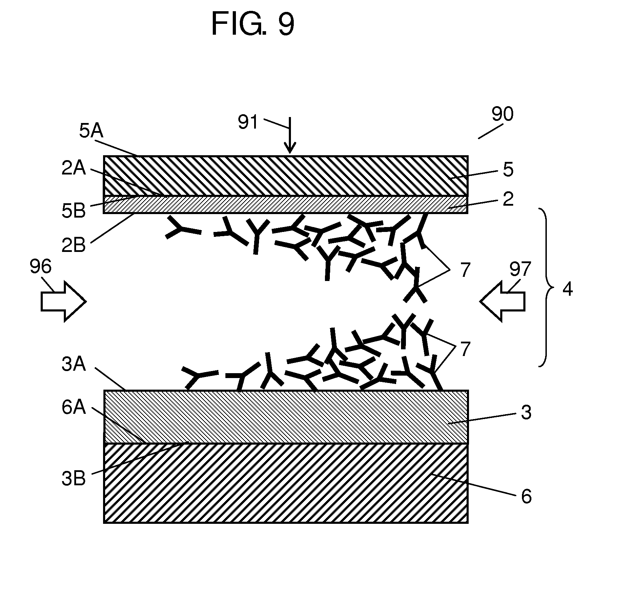

FIG. 9 is a sectional view of plasmon sensor 90 according to a fourth exemplary embodiment of the present invention. Plasmon sensor 90 differs from plasmon sensor 1 in the first exemplary embodiment in that capturing bodies 7 within hollow region 4 are disposed with an uneven density. Specifically, among specimen inserting sections 96 and 97 where a specimen of plasmon sensor 90 can be inserted, the density of disposed capturing bodies 7 is higher as getting closer to the specimen inserting section 97 side.

For example, when plasmon sensor 90 is used with the aim of detecting the existence or non-existence of an antigen in human saliva, the specimen inserting section 96 side is put into a mouth of a test subject, and the saliva is inserted into hollow region 4 by capillarity. By this usage method, it is possible to reduce extraction of a part of capturing bodies 7 into the mouth.

In another application, inserting the specimen, on the contrary, from specimen inserting section 97 can lead to efficient specific binding between capturing body 7 and analyte 8.

For realizing the configuration as in FIG. 9 where the density of disposed capturing bodies 7 within hollow region 4 is uneven, for example, such a method as follows can be applied. First, plasmon sensor 90 before physical adsorption of capturing bodies 7 is held in an inclined state such that the specimen inserting section 96 side is disposed above the specimen inserting section 97 side. A specimen containing capturing bodies 7 is then inserted from the specimen inserting section 97 side with an aid of capillarity. More capturing bodies 7 are distributed to the specimen inserting section 97 side due to gravitation. In this state, a medium of the specimen within hollow region 4 is dried or vaporized, the uneven density of disposed capturing bodies 7 shown in FIG. 9 can be realized.

FIFTH EXEMPLARY EMBODIMENT

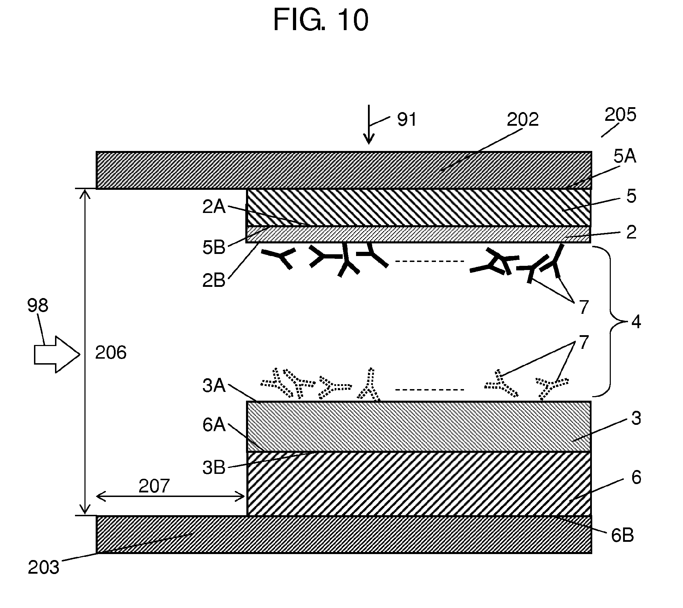

FIG. 10 is a sectional view of plasmon sensor 205 according to a fifth exemplary embodiment of the present invention. Plasmon sensor 205 differs from plasmon sensor 1 in the first exemplary embodiment in that holding section 202 is fixed to top surface 5A of holding section 5, and holding section 203 is fixed to bottom surface 6B of holding section 6. Further, capturing body 7 is not disposed in a region of holding section 202 which does not confront metal layer 2, and capturing body 7 is also not disposed in a region of holding section 203 which does not confront metal layer 3. As a result, capturing body 7 is not disposed in specimen inserting section 98.

For example, when plasmon sensor 205 is used with the aim of detecting the existence or non-existence of an antigen in human saliva, specimen inserting section 98 is put into the mouth of the test subject, and the saliva is inserted into hollow region 4 by capillarity. At that time, it is possible to reduce extraction of a part of capturing bodies 7 into the mouth.

Specimen inserting section 98 indicates a region surrounded by region 206 and region 207. Holding section 202 is formed of material with small attenuation of electromagnetic wave 91.

Although the configuration with holding sections 202 and 203 is shown in plasmon sensor 205, holding sections 202 and 203 may not be used, and holding sections 5 and 6 may be formed as shapes with larger areas than those of metal layers 2 and 3. Even with this configuration, a similar effect can be obtained.

Like plasmon sensor 90 of the fourth exemplary embodiment, plasmon sensor 205 may be configured such that the density of disposed capturing bodies 7 is uneven. It is thereby possible to obtain similar advantageous effects to plasmon sensor 90.

Further, particle 201 can be used for plasmon sensor 205 in a similar manner to plasmon sensor 81 of the third exemplary embodiment. It is thereby possible to obtain similar advantageous effects to that in the third exemplary embodiment.

Moreover, similarly to plasmon sensor 71 of the second exemplary embodiment, additive 200 may be disposed around capturing body 7 also in plasmon sensor 205. This allows plasmon sensor 205 to also exert similar advantageous effects to plasmon sensor 71.

Further, plasmon sensor 205 in the fifth exemplary embodiment can be manufactured, and can also be used, in similar manners to plasmon sensor 1. Further, plasmon sensor 205 also has similar advantageous effects to plasmon sensor 1 in the first exemplary embodiment.

In addition, although holding section 5 is disposed above metal layer 2 in the first to fifth exemplary embodiments, this is not restrictive, and it may be disposed below metal layer 2 as shown in FIG. 11. FIG. 11 is a sectional view of another plasmon sensor according to an exemplary embodiment of the present invention.

In the case of holding section 5 being disposed below, capturing bodies 7 are disposed on the bottom surface of holding section 5. When holding section 5 has a high relative dielectric constant, the resonance wavelength can be set long, whereby it is possible to make lower the frequency of the electromagnetic wave supplied from the above of metal layer 2, and further to reduce cost of the electromagnetic wave source. As described above, when holding section 5 is disposed below metal layer 2, holding section 5 is preferably formed of material having a low dielectric constant and a low loss.

Further, although metal layer 2, holding section 5, metal layer 3, and holding section 6 are shown in flat shape in first to fifth exemplary embodiments, this is not restrictive, and even with an uneven shape, a similar effect can be obtained. Accordingly, even if fine unevenness occurs in the manufacturing process, they function as the plasmon sensor without any problem. Capturing body 7 may be a receptor, an aptamer or the like other than the antibody.

Moreover, although the cases of using light as the electromagnetic wave are mainly described, even when an electromagnetic wave having a wavelength other than light is used, a similar effect can be obtained. In that case, by making holding section 5 formed of nonmetal material such as glass, holding section 5 can transmit an electromagnetic wave therethrough.

INDUSTRIAL APPLICABILITY

The plasmon sensor in the present invention has a small-sized, simple configuration, and is thus usable for a small-sized, low-cost biosensor, and the like.

REFERENCE MARKS IN THE DRAWINGS

| 1, 71, 81, 90, 205 | plasmon sensor |
| 2 | first metal layer (metal layer) |
| 2A, 3A | top surface |
| 2B, 3B | bottom surface |
| 3 | second metal layer (metal layer) |
| 4 | hollow region |
| 5, 6 | holding section |
| 5A, 6A | top surface |
| 5B, 6B | bottom surface |
| 7 | analyte capturing body (capturing body) |
| 8 | analyte |
| 9 | antibody |
| 61 | medium |
| 62 | specimen |
| 91, 93, 591, 593 | electromagnetic wave |
| 92 | electromagnetic wave source |
| 94 | sensing section |
| 95 | region |

| 95A, 95B | region |
| 96, 97, 98 | specimen inserting section |
| 200 | additive |
| 201 | particle |
| 202, 203 | holding section |
| 206, 207 | region |
| 501, 502, 505, 506 | analysis model |
| 501N | normal line direction |
| 508 | resultant substance |

**Claims**

1. A plasmon sensor comprising:

   a first metal layer having a bottom surface and a top surface configured to be supplied with an electromagnetic wave; and
   a second metal layer having a top surface confronting the bottom surface of the first metal layer, wherein
   a hollow region configured to be filled with a specimen containing a medium is provided between the first metal layer and the second metal layer, and
   analyte capturing bodies are physically adsorbed to at least one below of the first metal layer and above of the second metal layer.

2. A plasmon sensor comprising:

   a first metal layer, having a bottom surface and a top surface configured to be supplied with an electromagnetic wave; and
   a second metal layer, having a top surface confronting the bottom surface of the first metal layer, wherein
   a hollow region configured to be filled with a specimen containing a medium is provided between the first metal layer and the second metal layer,
   analyte capturing bodies are disposed at least one of below the first metal layer and above the second metal layer, and
   the analyte capturing bodies are not oriented.

3. The plasmon sensor according to any one of claims 1 and 2, wherein
   particles are disposed between the first metal layer and the second metal layer, and
   the analyte capturing bodies are chemically adsorbed to surfaces of the particles.

4. The plasmon sensor according to claim 3, wherein
   the particles are made of metal.

5. The plasmon sensor according to claim 3, wherein
   the particles are made of dendrimer.

6. The plasmon sensor according to any one of claims 1 and 2, further comprising
   an additive physically adsorbed together with the analyte capturing bodies.

7. The plasmon sensor according to any one of claims 1 and 2, wherein
   the analyte capturing bodies are disposed with an uneven density.

8. The plasmon sensor according to any one of claims 1 and 2, further comprising:

a specimen inserting section for insertion of a specimen containing an analyte into the hollow region, wherein the analyte capturing bodies are not disposed in the specimen inserting section.

9. A method for using a plasmon sensor, the plasmon sensor comprising:

a first metal layer having a top surface and a bottom surface,
a second metal layer having a top surface confronting the bottom surface of the first metal layer, wherein a hollow region is provided between the first metal layer and the second metal layer, and
analyte capturing bodies are physically adsorbed at least one below the first metal layer and above the second metal layer,

the method comprising:

inserting a specimen into the hollow region with an aid of capillarity;
supplying an electromagnetic wave to a top surface side of the first metal layer; and
sensing at least one of a change in amplitude of an electromagnetic wave reflected or radiated from the top surface of the first metal layer and a change in resonance wavelength.

10. A method for manufacturing a plasmon sensor, the method comprising:

preparing a plasmon sensor structure which has a first metal layer having a bottom surface and a top surface configured to be supplied with an electromagnetic wave, and a second metal layer having a top surface confronting the bottom surface of the first metal layer, and in which a hollow region is provided between the first metal layer and the second metal layer;
inserting a medium containing analyte capturing bodies into the hollow region with an aid of capillarity; and
drying the medium after insertion of the analyte capturing bodies into the hollow region so as to dispose the analyte capturing bodies at least one of below the first metal layer and above the second metal layer.

FIG. 1

FIG. 2

# FIG. 3

# FIG. 4

# FIG. 5A

# FIG. 5B

## FIG. 6A

## FIG. 6B

# FIG. 7

# FIG. 8

# FIG. 9

# FIG. 10

# FIG. 11

# FIG. 12

<table>
<tr><td colspan="2">**INTERNATIONAL SEARCH REPORT**</td><td>International application No.</td></tr>
<tr><td colspan="2"></td><td>PCT/JP2011/002567</td></tr>
</table>

**A. CLASSIFICATION OF SUBJECT MATTER**
*G01N21/27*(2006.01)i

According to International Patent Classification (IPC) or to both national classification and IPC

**B. FIELDS SEARCHED**

Minimum documentation searched (classification system followed by classification symbols)
G01N21/00-21/61

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y<br>A | JP 09-257702 A (Toto Ltd.),<br>03 October 1997 (03.10.1997),<br>paragraphs [0004], [0013] to [0016]; fig. 1<br>(Family: none) | 1,2,7-10<br>6<br>3-5 |
| Y | JP 2009-150708 A (Canon Inc.),<br>09 July 2009 (09.07.2009),<br>paragraph [0021]<br>(Family: none) | 6 |
| A | JP 02-297053 A (Amersham International PLC.),<br>07 December 1990 (07.12.1990),<br>entire text; all drawings<br>& GB 8909702 A      & GB 8909702 A0<br>& EP 395222 A2      & CA 2014405 A | 1-10 |

[X] Further documents are listed in the continuation of Box C.      [ ] See patent family annex.

| | |
|---|---|
| *  Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>12 July, 2011 (12.07.11) | Date of mailing of the international search report<br>26 July, 2011 (26.07.11) |
|---|---|
| Name and mailing address of the ISA/<br>Japanese Patent Office | Authorized officer |
| Facsimile No. | Telephone No. |

Form PCT/ISA/210 (second sheet) (July 2009)

**INTERNATIONAL SEARCH REPORT**

International application No.

PCT/JP2011/002567

| C (Continuation). DOCUMENTS CONSIDERED TO BE RELEVANT | | |
|---|---|---|
| Category* | Citation of document, with indication, where appropriate, of the relevant passages | Relevant to claim No. |
| A | JP 10-019769 A (Fuji Photo Film Co., Ltd.), 23 January 1998 (23.01.1998), entire text; all drawings & US 5856873 A & US 5907408 A & US 5917608 A & US 5926284 A & EP 805347 A2 & EP 1650547 A2 & EP 1650548 A2 & EP 1650549 A2 & EP 1650550 A2 | 1-10 |

Form PCT/ISA/210 (continuation of second sheet) (July 2009)

**INTERNATIONAL SEARCH REPORT**

| International application No. |
|---|
| PCT/JP2011/002567 |

**Box No. II      Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:
   See extra sheet.

1. ☐ As all required additional search fees were timely paid by the applicant, this international search report covers all searchable claims.

2. ☒ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**        ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

| **INTERNATIONAL SEARCH REPORT** | International application No. |
| --- | --- |
| | PCT/JP2011/002567 |

Continuation of Box No.III of continuation of first sheet(2)

In paragraphs [0004] and [0013] - [0016] and [fig. 1] of JP 09-257702 A (as will be called "document 1"), there is disclosed an invention of a surface plasmon resonance sensor device caused to face the inside of a cell having an entrance and an exit, comprising: a metal thin film (corresponding to the "first metal layer" of claim 1); and a counter electrode (corresponding to the "second metal layer" of claim 1), wherein an antibody (corresponding to the "analyte trap" of claim 1) for adsorbing protein molecules in a sample solution uniquely is fixed on the surface of the metal thin film, and wherein a sample liquid is caused to flow in said cell while the metal thin film is being irradiated with a light (corresponding to the "electromagnetic waves" of claim 1) and while a voltage is being applied between the metal thin film and the counter electrode, so that protein molecules are attracted to the side of the metal thin film and are excellently adsorbed by an antibody on the metal thin film, whereby the protein molecules are sensitively detected by measuring the intensity (or amplitude) or the resonance angle of the reflected light which has caused a surface plasmon resonance on the metal thin film. In the invention disclosed in document 1, moreover, the antibody exists on the metal thin film. It is, therefore, apparent that the aforementioned antibody exists in an offset density distribution in the area between the metal thin film and the counter electrode. It is also apparent from [fig. 1] of document 1 that no antibody is disposed in the entrance and exit (corresponding to a "sample introduction section" of claim 8) of the cell.

Thus, there is no constitutional difference between the invention of claims 1, 7 and 8 of the present application and the invention disclosed in document 1. Thus, the invention of the aforementioned claims is not admitted to involve any novelty to and any special technical feature over the invention disclosed in document 1.

Hence, the present application is admitted to involve two or more inventions containing a main invention of claims 1 - 8.

Form PCT/ISA/210 (extra sheet) (July 2009)

**EP 2 570 797 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005181296 A